Europäisches Patentamt

European Patent Office

Office européen des brevets

(19)

(11) EP 1 588 762 A1

(12) EUROPEAN PATENT APPLICATION
published in accordance with Art. 158(3) EPC

(43) Date of publication:
26.10.2005 Bulletin 2005/43

(51) Int Cl.7: B01J 29/89, B01J 35/10,
C07D 301/19, C07D 303/04

(21) Application number: 03778946.8

(86) International application number:
PCT/JP2003/016073

(22) Date of filing: 16.12.2003

(87) International publication number:
WO 2004/056476 (08.07.2004 Gazette 2004/28)

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 19.12.2002 JP 2002367818

(71) Applicant: Sumitomo Chemical Company,
Limited
Chuo-ku, Tokyo 104 (JP)

(72) Inventor: YAMAMOTO, Jun
Sodegaura-shi, Chiba 299-0241 (JP)

(74) Representative: Lips, Hendrik Jan George
HAAGSCH OCTROOIBUREAU
Breitnerlaan 146
2596 HG Den Haag (NL)

(54) **METHOD FOR PRODUCING TITANIUM-CONTAINING SILICON OXIDE CATALYST**

(57)     A process for producing a titanium-containing silicon oxide catalyst satisfying conditions (1) to (3);

(1) an average pore diameter is 10 Å or more,
(2) 90% or more of the total pore volume has a pore diameter of 5 to 200Å , and
(3) a specific pore volume is 0.2 cm$^3$/g or more,

which comprises the following steps:

first step of obtaining a solid containing a catalyst component and a template by mixing and stirring a silica source, a titanium source and a quaternary ammonium ion as a template in a liquid state; second step of removing the template from the solid obtained in the first step by solvent extraction; third step of substituting the solvent used for the extraction, which was contained in the solid after the removal of the template with a solvent which is substantially inert to a silylating agent to be used in the fourth step; and fourth step of obtaining a silylated catalyst by subjecting the solid obtained in the third step to silylation.

EP 1 588 762 A1

**Description**

**TECHNICAL FIELD**

The present invention relates to an efficient process for producing a titanium-containing silicon oxide catalyst. More particularly, the present invention relates to an efficient process for producing a titanium-containing silicon oxide catalyst which can be used for reaction of a hydroperoxide with an olefin type compound for obtaining an oxirane compound, and which can exhibit high activity.

**BACKGROUND ART**

Processes of obtaining an oxirane compound from an olefin type compound and a hydroperoxide in the presence of a catalyst, are publicly known. As the catalyst used herein, for example, U.S.Patent No.4,367,342 discloses a specified titanium-supported silicon oxide catalyst. However, it was difficult to say that the conventional catalyst is sufficiently satisfied from the viewpoint of realization of higher activity.

On the other hand, though producing processes of catalysts having high activity and high selectivity for obtaining an oxirane compound from a hydroperoxide and an olefin type compound, are known (e.g. US 5,783,167, JP 7-300312, US 6,096,910, US 6,211,388, JP 2002-224563, JP 2002-239381), these processes were not sufficient from the

viewpoint of productivity of the catalyst. **DISCLOSURE OF THE INVENTION**

The present invention provides a process for producing a titanium-containing silicon oxide catalyst which can be used for reaction obtaining, for example, an olefin oxide from a hydroperoxide and an olefin type compound, and which can exhibit high activity.

Namely, the present invention relates to an efficient process for producing a titanium-containing silicon oxide catalyst satisfying all of the following conditions (1) to (3);

(1) an average pore diameter is 10Å or more,
(2) 90% or more of the total pore volume has a pore diameter of 5 to 200Å, and
(3) a specific pore volume is 0.2 cm$^3$/g or more, which comprises the following first to fourth steps:

first step: a step of obtaining a solid containing a catalyst component and a template by mixing and stirring a silica source, a titanium source and a quaternary ammonium ion as a template in a liquid state;
second step: a step of removing the template from the solid obtained in the first step by solvent extraction;
third step: a step of substituting the solvent used for the extraction which was contained in the solid after the removal of the template, with a solvent which is substantially inert to a silylating agent to be used in the following fourth step; and
fourth step; a step of obtaining a silylated catalyst by subjecting the solid obtained in the third step to silylation.

**MODE FOR CARRYING OUT THE INVENTION**

The catalyst obtained by the present invention is a titanium-containing silicon oxide catalyst satisfying all of the following conditions (1) to (3);

(1) an average pore diameter is 10Å or more,
(2) 90% or more of the total pore volume has a pore diameter of 5 to 200Å, and
(3) a specific pore volume is 0.2 cm$^3$/g or more.

Herein, the specific pore volume means pore volume per 1 g of the catalyst.

Measurements of the above-described conditions (1) to (3) can be conducted by ordinary methods such as a physical adsorption method using gas such as nitrogen, argon or the like.

The catalyst of the present invention may or may not have a peak showing an interplanar spacing (d) in a X-ray diffraction (XRD). Herein, the peak showing an interplanar spacing (d) means a peak derived from the crystallinity and regularity of a solid, and a broad peak derived from an amorphous part may exist.

The catalyst obtained in the present invention preferably has an absorption peak in the region of $960 \pm 5$ cm$^{-1}$ in the infrared absorption spectrum from the viewpoint of high activity. This peak is assumed to correspond to that of titanium introduced into the silica skeleton.

The catalyst is produced by a process having the following steps:

> first step: a step of obtaining a solid containing a catalyst component and a template by mixing and stirring a silica source, a titanium source and a quaternary ammonium ion as a template in a liquid state;
> second step: a step of removing the template from the solid obtained in the first step by solvent extraction;
> third step: a step of substituting the solvent used for the extraction, which was contained in the solid after the removal of the template with a solvent which is substantially inert to a silylating agent to be used in the following fourth step; and
> fourth step; a step of obtaining a silylated catalyst by subjecting the solid obtained in the third step to silylation.

The first step is a step of obtaining a solid containing a catalyst component and a template by mixing and stirring a silica source, a titanium source and a quaternary ammonium ion as the template in a liquid state.

When a reagent to be used is solid, it is preferable to be used as a solution in which it is dissolved or dispersed in a solvent.

The silica source includes amorphous silica and alkoxysilane such as tetramethyl orthosilicate, tetraethyl orthosilicate and tetrapropyl orthosilicate. A silica source having an organic group such as alkytrialkoxysilane, dialkyldialkoxysilane and 1,2-bis(trialkoxysilyl)alkane, can be used. These can be used alone, or may be used as a mixture of two or more kinds.

The titanium source includes titanium alkoxides such as tetramethyl titanate, tetraethyl titanate, tetrapropyl titanate, tetraisopropyl titanate, tetrabutyl titanate, tetraisobutyl titanate, tetra-2-ethylhexyl titanate, tetraoctadecyl titanate; titanium(IV) oxyacetylacetonate, titanium(IV) diisopropoxybisacetylacetonate, and the like; titanium halides, for example, titanium tetrachloride, titanium tetrabromide and titanium tetraiodide; titanyl sulfate; and the like.

As the template, any one of cationic surfactants such as alkylammoniums, dialkylammoniums, trialkylammoniums and benzylammoniums, anionic surfactants such as alkylsulfate ions and alkylphosphate ions and nonionic surfactants such as polyalkyleneoxides and alkylamines, can be applied. Among them, a quaternary ammonium ion represented by the general formula (I) is suitably used.

$$[NR^1R^2R^3R^4]^+ \tag{I}$$

(wherein, $R^1$ represents a linear or branched hydrocarbon group having 2 to 36 carbon atoms, and $R^2$ to $R^4$ represent an alkyl group having 1 to 6 carbon atoms).

$R^1$ is a linear or branched hydrocarbon group having 2 to 36 carbon atoms, preferably 10 to 18 carbon atoms. $R^2$ to $R^4$ are an alkyl group having 1 to 6 carbon atoms, and preferably each of $R^2$ to $R^4$ is a methyl group. Specific examples of the quaternary ammonium ion represented by the general formula(I) include cations such as hexadecyltrimethylammonium, dodecyltrimethylammonium, benzyltrimethylammonium, dimethyldidodecylammonium and hexadecylpyridinium. Further, these quaternary ammonium ions represented by the general formula(I) can be used alone or may be used as a mixture of two or more kinds.

Examples of the solvent include water and alcohols such as methanol, ethanol, n-propanol, 2-propanol, n-butanol, sec-butanol, t-butanol, vinyl alcohol, allyl alcohol, cyclohexanol and benzyl alcohol, and diols, mixtures thereof and the like. The amount used of the titanium source based on the silica source is preferably from $10^{-5}$ to 1, more preferably from 0.00008 to 0.4 in terms of molar ratio. The amount used of the quaternary ammonium ion based on the total amounts of silica source and titanium source is preferably from $10^{-2}$ to 2 in terms of molar ratio. Further, for promoting the reaction of the silica source with the titanium source, it is preferable to impart alkalinity or acidity to the mixed solution. As the alkali source, a quaternary ammonium hydroxide is preferable, and examples thereof include tetramethylammonium hydroxide, tetraethylammonium hydroxide and tetrapropylammonium hydroxide, and a hydroxide of the quaternary ammonium ion represented by the general formula (I) is more preferably used. In addition, examples of the acid include inorganic acids such as hydrochloric acid, sulfuric acid and nitric acid, and organic acids such as formic acid, acetic acid and propionic acid.

The mixing and stirring temperature is usually from -30 to 100°C. A solid is formed by mixing and stirring, and the solid may be aged for further growth thereof. The aging time is usually 180 hours or less, and the aging temperature is usually from 0 to 200°C. When heating is required in aging, it is preferable that the mixture is transferred into a pressure vessel and heating is conducted in a closed pressure vessel for avoiding vaporization of the solvent.

The second step is a step of removing the template from the solid by extraction. The extractive removal of the template can be easily accomplished by subjecting the solid containing a catalyst component and the template obtained in the first step to solvent extraction.

A technique for extracting a template with a solvent is reported by Whitehurst et al.(see U.S.Patent 5,143,879).

The solvent used in extraction may include solvents which can dissolve a compound used as the template, and oxa- and/or oxo-substituted hydrocarbons having carbon atoms of 1 to about 12 in a liquid state at room temperature can be generally used. Suitable examples of such solvents include alcohols, ketones, (acyclic and cyclic)ethers and esters. Examples thereof include hydroxy-substituted hydrocarbons such as methanol, ethanol, ethylene glycol, propylene glycol, isopropanol, n-butanol and octanol; oxo-substituted hydrocarbons such as acetone, diethyl ketone, methyl ethyl ketone and methyl isobutyl ketone; ethers of a hydrocarbon such as diisobutyl ether and tetrahydrofuran; esters of hydrocarbons such as methyl acetate, ethyl acetate, butyl acetate and butyl propionate; and the like. But, alcohols are preferable from the viewpoint of solubility to the template, among them, methanol is further preferable. The weight ratio of the solvent to the solid containing the catalyst component and the template is usually from 1 to 1000, preferably from 5 to 300. For improving efficiency of the extraction, an acid or salt thereof may be added to these solvents.

Examples of the acid used include inorganic acids such as hydrochloric acid, sulfuric acid, nitric acid and bromic acid, and organic acids such as formic acid, acetic acid and propionic acid. Examples of the salt thereof include alkali metal salts, alkaline earth metal salts, ammonium salts and the like.

The concentration in the solvent of the acid or the salt thereof to be added is preferably 10 mol/l or less, further preferably 5 mol/l or less. When the concentration in the solvent of the acid or the salt to be added is too high, the catalytic activity may be lowered by elution of titanium existing in the catalyst.

After adequately mixing a solid containing the catalyst component and the template with a solvent, the liquid portion is separated by a method such as filtration or decantation. This operation is repeated required times. Further, a method of filling the solid containing the catalyst component and the template in a reaction tube, then, of flowing the extraction solvent through there is also possible. The completion of the solvent extraction can be found by, for example, analyzing the liquid portion. The extraction temperature is preferably 0 to 200°C, further preferably 20 to 100°C. When the boiling point of the extraction solvent is too low, the extraction may be carried out under pressure.

The quaternary ammonium ion represented by the general formula I) obtained after the extraction can be reused as the template material in the first step after recovery. Further, similarly, the extraction solvent can be also reused after purification through ordinary distillation or the like.

The third step is a step of substituting the solvent used for the extraction, which was contained in the solid after the removal of the template with a solvent which is substantially inert to a silylating agent to be used in the fourth step. The feature of the present invention is to contain this step.

The extraction solvent contained in the solid after the extraction of the template is usually removed by a drying operation because alcohols used suitably for removal of a template in a publicly known invention, retard from the objective reaction through a reaction with a silylating agent in the silylation of the next step.

As a drying apparatus, a conical or shelves dryer equipped with a fan heater or vacuum device is listed. However, in carrying out the drying of them economically and efficiently, the drying requires a lot of time, therefore, it was not sufficient from the viewpoint of productivity of the catalyst. In addition, the catalytic performance was sometimes deteriorated since shrinkage of micro pores of the catalyst and modification of the catalyst surface occurred depending on conditions of drying.

For achievement of the present invention which is to produce the catalyst efficiently, the extraction solvent contained in the solid obtained in the second step, is substituted with a solvent substantially inert to the silylating agent used in the following fourth step described below.

The substituting solvent used in the substitution step may be one fulfil requirements for which it is substantially inert to the silylating agent and can dissolve the extraction solvent used in the second step.

The solvent suitably used in the substitution operation, usually includes hydrocarbons, halogenated hydrocarbons, ketones, ethers, esters, N,N-di-substituted amides, nitriles and tertiary amines, having 1 to about 12 carbon atoms and being liquid at normal temperature, and, for example, hexane, cyclohexane, chloroform, benzene, toluene, xylene, acetone, diethyl ketone, methyl ethyl ketone, methyl isobutyl ketone, diethyl ether, diisobutyl ether, tetrahydrofuran, dioxane, methyl acetate, ethyl acetate, dimethyl formamide, acetonitrile, pyridine, triethylamine and dimethyl sulfoxide are listed. From the relation to the subsequent fourth step, preferable solvent for substitution is the hydrocarbons, and among these, toluene is more preferable.

These solvents can be used alone, and can be also used as a solution in which two or more of solvents are mixed.

In the substitution operation, after a solvent for substitution and the solid containing the extraction solvent obtained in the second step were sufficiently mixed, its liquid phase part is separated by a method of filtration, decantation or the like. This operation is properly repeated.

Further, it is also possible to substitute the solvent by a method of packing the solid containing the extraction solvent into a reaction tube or the like, then flowing the solvent for substitution through there. From the viewpoint of productivity of the catalyst, it is preferable to conduct the second and third steps in the present invention and further the fourth step, in the same reactor. The completion of the substitution operation can be found by, for example, analysis of the liquid phase part. The substitution temperature is preferably 0 to 200°C, further preferably 20 to 100°C. When the boiling point of the solvent used in the substitution operation is low, the substitution may be carried out under pressure.

In addition, the solvent for substitution used in this step, can be reused after the extraction solvent was removed by a usual method such as distillation or extraction.

The fourth step is a step for obtaining a silylated catalyst by subjecting the solid obtained in the third step to silylation treatment.

The silylation may be carried out by gas phase method in which the solid obtained in the third step is reacted with gaseous silylating agent, or a liquid phase method in which the solid is reacted with the silylating agent in a solvent, further the liquid phase method is more preferable in the present invention. Usually, when the silylation is carried out by the liquid phase method, hydrocarbons are suitably used. Though the solvent for silylation is not necessarily the same as the substitution solvent used in the third step, the same solvent each other is preferable from the viewpoint of reuse of the solvent.

Examples of the silylation agent include organic silanes, organic silylamines, organic silylamides and derivatives thereof, organic silazanes and other silylation agents.

Examples of the organic silane include chrolotrimethylsilane, dichlorodimethylsilane, chlorobromodimethylsilane, nitrotrimethylsilane, chlorotriethylsilane, iododimethylbutylsilane, chlorodimethylphenylsilane, chlorodimethylsilane, dimethyl-n-propylchlorosilane, dimethylisopropylchlorosilane, t-butyldimethylchlorosilane, tripropylchlorosilane, dimethyloctylchlorosilane, tributylchlorosilane, trihexylchlorosilane, dimetylethylchlorosilane, dimethyloctadecylchlorosilane, n-butyldimethylchlorosilane, bromomethyldimethylchlorosilane, chloromethyldimethylchlorosilane, 3-chloropropyldimethylchlorosilane, dimethoxymethylchlorosilane, methylphenylchlorosilane, triethoxychlorosilane, dimethylphenylchlorosilane, methylphenylvinylchlorosilane, benzyldimethylchlorosilane, diphenylchlorosilane, diphenylmethylchlorosilane, diphenylvinylchlorosilane, tribenzylchlorosilane, 3-cyanopropyldimethylchlorosilane.

Examples of the organic silylamine include N-trimethylsilylimidazole, N-t-butyldimethylsilylimidazole, N-dimethylethylsilylimidazole, N-dimethyl-n-propylsilylimidazole, N-dimethylisopropylsilylimidazole, N-trimethylsilyldimethylamine, N-trimethylsilyldiethylamine, N-trimethylsilylpyrrole, N-trimethylsilylpyrrolidine, N-trimethylsilylpiperidine, 1-cyanoethyl (diethylamino) dimethylsilane, pentafluorophenyldimethylsilylamine.

Examples of the organic silylamide and derivatives include N,O-bistrimethylsilylacetamide, N,O-bistrimethylsilyltrifluoroacetamide, N-trimethylsilylacetamide, N-methyl-N-trimethylsilylacetamide, N-methyl-N-trimethylsilyltrifluoroacetamide, N-methyl-N-trimethylsilylheptafluorobutylamide, N-(t-butyldimethylsilyl)-N-trifluoroacetamide, N,O-bis(diethylhydrosilyl)trifluoroacetamide.

Examples of the organic silazane include hexamethyldisilazane, heptamethyldisilazane, 1,1,3,3-tetramethyldisilazane, 1,3-bis(chloromethyl)tetramethyldisilazane, 1,3-divinyl-1,1,3,3-teteramethyldisilazane, 1,3-diphenyltetramethyldisilazane.

Examples of the other silylation agent include N-methoxy-N,O-bistrimethylsilyltrifluoroacetamide, N-methoxy-N,O-bistrimethylsilyl carbamate, N,O-bistrimethylsilyl sulfamate, trimethylsilyltrifluoromethane sulfonate, N,N'-bistrimethylsilylurea. The preferable silylation agent is hexamethyldisilazane.

The catalyst of the present invention is usually used as a molded catalyst via a step for molding a solid containing the catalyst component. Though the molding step may be conducted in any stage, before or after the above-mentioned template-removing step, or after the silylation step, it is preferable to conduct before the template-removing step from the viewpoint of suppression of degradation of catalyst properties such as specific surface area, pore volume and the like. As the molding method, any method such as compression molding, extrusion molding or the like may be used. An organic or inorganic binder usually used can be used in the extrusion molding, but lowering of catalyst activity may be caused by addition of the binder. In the production of the molded catalyst, the compression molding is the most preferable from the viewpoint of strength and physical properties of the catalyst.

As the compression molding, a roll press molding (briquetting, compacting), oil hydraulic press molding, tabletting and the like can be listed. The pressure in compression is usually 0.1 to 10 ton/cm$^2$, preferably 0.2 to 5 ton/cm$^2$, further preferably 0.5 to 2 ton/cm$^2$. When the pressure is too low, the strength of a molded body is sometimes inadequate. On the other hand, when the pressure is too high, the physical properties of the catalyst sometimes become inadequate because pores are broken. In carrying out the compression molding, it is preferable that a solid containing a catalyst component contains water in a proper amount, and a molded body having a sufficient strength can be produced under lower pressure by this. The water content of the material to be subjected to the compression molding is preferably 1 to 70 % by weight, further preferably 5 to 40 % by weight. The water amount may be adjusted by a dryness degree during drying of a wet solid, and may be adjusted by adding water to an adequately dried solid.

In addition, a binder usually used or the like may be added within a range of no obstacle to a desired performance.

The shape of the molded body may be any shape such as tablet, sphere or ring. The molded body may be used as it is or after pulverizing to a proper size.

The catalyst of the present invention can be used for selective oxidation, for example, in addition to epoxidation of an olefin type compound, various oxidation reactions of organic compounds because the catalyst has a large surface area and highly dispersed titanium active sites. Further, if desired, it is also possible to increase acid sites of the catalyst with addition of a third component such as alumina, etc. , and the catalyst can be used for alkylation, catalytic reforming, etc.

The catalyst of the present invention can be optimally can be used for production of an oxirane compound in which an olefin type compound is reacted with a hydroperoxide, in particular.

The olefin type compound may be acyclic, mono-cyclic, di-cyclic or poly-cyclic compounds, and mono-olefin type, di-olefin type or poly-olefin type compounds. When the number of olefin bonds is two or more, these may be a conjugated bond or non-conjugated bond. Olefin type compounds having 2 to 60 carbon atoms are usually preferred. These may have a substituent, and the substituent is preferably a relatively stable substituent. Examples of such the hydrocarbon include ethylene, propylene, 1-butene, isobutylene, 1-hexene, 2-hexene, 3-hexene, 1-octane, 1-decene, styrene and cyclohexene. Apt examples of the di-olefin type compound include butadiene and isoprene. A substituent may exist, and as the example thereof, a halogen atom is listed, further various substituents containing an oxygen, sulfur or nitrogen atom together with a hydrogen and/or carbon atom, may exist. A particularly preferable olefin type compound is an olefin type unsaturated alcohol and an olefin type unsaturated hydrocarbon substituted with a halogen, and, as examples thereof, allyl alcohol, crotyl alcohol and allyl chloride are listed. Particularly preferable compound is an alkene having 3 to 40 carbon atoms, and this compound may be substituted with a hydroxy group or a halogen atom.

As examples of a hydroperoxide, organic hydroperoxides can be listed.

The organic hydroperoxide is a compound represented by the general formula;

$$R\text{-}O\text{-}O\text{-}H$$

(R represents a hydrocarbyl group), and is reacted with an olefin type compound to produce an oxirane compound and a compound, R-OH. Preferably, the group R is a group having 3 to 20 carbon atoms. Most preferably, this is a hydrocarbyl group having 3 to 10 carbon atoms, and particularly a secondary or tertiary alkyl group or aralkyl group. Among them, tertiary alkyl groups, and secondary or tertiary aralkyl groups are particularly preferable, and specific examples thereof include a tertiary butyl group, tertiary pentyl group, cyclopentyl group and 2-phenyl-2-propyl group. Further, various tetralinyl groups formed by eliminating hydrogen from an aliphatic side chain of a tetralin molecule, are also listed.

When cumene hydroperoxide as the organic hydroperoxide is used, the resulting hydroxyl compound is 2-phenyl-2-propanol. This can be converted to $\alpha$-methyl styrene by dehydration reaction. $\alpha$-methyl styrene is a industrially useful substance.

Tert-amylene produced by dehydration of tert-pentyl alcohol obtained by using tert-pentyl hydroperoxide as the organic hydroperoxide, is a useful substance as a precursor of isoprene. Tert-pentyl alcohol is also useful as a precursor of methyl tert-pentyl ether which is an octane booster.

Tert-butyl alcohol obtained by using t-butyl hydroperoxide as an organic hydroperoxide is useful as a precursor of methyl tert-butyl ether which is an octane booster.

Hydrogen peroxide can be listed as an example other than organic hydroperoxides.

Hydrogen peroxide is a compound represented by the chemical formula, HOOH, and can be obtained usually in the form of an aqueous solution. It reacts with an olefin type compound to produce an oxirane compound and water.

The organic hydroperoxide and hydrogen peroxide, which are used as a raw material, may be a thin or dense purified or non-purified material.

The epoxidation can be carried out in a liquid phase by using a solvent and/or a diluent. The solvent and diluent are a substance which are liquid under the pressure and temperature under which the reaction is conducted, and should be substantially inert against the reactants and products. The solvent may be a substance existing in the hydroperoxide solution to be used. For example, when cumene hydroperoxide is a mixture of cumene hydroperoxide and cumene which is a raw material thereof, said cumene can be used as a substitute for the solvent without especially adding a solvent.

The epoxidation temperature is usually from 0 to 200°C , preferably from 25 to 200°C. The pressure may be a pressure enough to keep the reaction mixture liquid. Usually, the pressure is advantageously from 100 to 10000 kPa.

After completion of the epoxidation, a liquid mixture containing desired product can be easily separated from a catalyst composition. Next, the liquid mixture can be purified by a suitable method. Purification includes fractional distillation, selective extraction, filtration, washing and the like. The solvent, catalyst, non-reacted propylene and non-reacted hydroperoxide can be used again by recycling.

The reaction in which the catalyst of the present invention is used, can be carried out in the form of a slurry or a fixed bed, and, in the case of a large scale of industrial operation, it is preferable to use a catalyst in the form of a fixed bed. The present process can be carried out by a batchwise method, semi-continuous method or continuous method. When a solution containing a reactant is introduced through a fixed bed, a liquid mixture obtained from the reaction zone does not contain catalyst at all or contains substantially no catalyst.

**EXAMPLE**

The present invention is illustrated by the following

Examples.

Example 1

Preparation of catalyst powder

While 125.1 parts by weight of 16 wt% hexadecyltrimethyl ammonium hydroxide aqueous solution were stirred, a mixed solution of 1.85 parts by weight of tetraisopropyl orthotitanate and 10.0 parts by weight of 2-propanol was added dropwise to this at room temperature. After stirring of 30 minutes, 38.1 parts by weight of tetramethyl orthosilicate were added dropwise. Thereafter, stirring was continued at room temperature for 3 hours, and thus obtained precipitate was filtered. The precipitate obtained was dried at 70°C under vacuum.

Preparation of molded body

Water was added to 10.0 parts by weight of a white solid obtained by the drying, with a sprayer so that the water content became 1.3 parts by weight, then mixed thoroughly. The resultant was subjected to compression-molding with a roll pressing machine. Thus obtained solid was pulverized, then molded catalyst of 1.0 to 2 . 0 mm was obtained using sieves. Solid less than 1.0 mm was recycled to conduct compression-molding again.

Extraction removal of template

Next, 150 g of the molded body obtained as described above was packed in a glass column of an inside diameter of 20 mm$\phi$ (sheath tube outside diameter; 6 mm $\phi$) to height of 1 m, then, (1) 1500 ml of methanol under room temperature, (2)a mixed solution of 3000 ml of methanol with 60 g of concentrated hydrochloric acid(content: 36 % by weight), (2) a mixed liquid of 100 ml of methanol with 1.0 g of concentrated hydrochloric acid under heating of 45°C, and (3) 3000 ml of methanol under heating of 45°C, in this order, were passed upwardly through the column at an LHSV of 6 h$^{-1}$. After completion of the flowing of the liquid, methanol in the column was drained from the lower part thereof.

Substitution of extraction solvent

Subsequently, 3000 ml of toluene heated to 80°C was passed upwardly through the column after drained, at an LHSV of 6h$^{-1}$ to substitute methanol with toluene. The time required for the substitution was 1 hour and 40 minutes. Then, toluene in the column was drained from the lower part thereof.

Silylation

After the substitution treatment described above, a mixed solution of 40 g of hexamethyldisilazane and 600 g of toluene was circulated through the column for 3 hours under heating of 110°C to carry out silylation. After removing the liquid, the molded catalyst was dried by flowing nitrogen at a rate of 1500 ml/minute under heating of 120°C for 3 hours. The obtained molded catalyst had an average pore diameter of 35 Å, a pore diameter of 5 to 200Å in 96% of the total pore volume and a specific pore volume of 0.79 cm$^3$/g.

Synthesis of propylene oxide(PO)

The molded catalyst obtained as described above was evaluated with a batch reaction apparatus (autoclave) using 25 % of cumene hydroperoxide(CHPO) and propylene(C3'). The catalyst of 1.0 g, 30.0 g of CHPO and 16.6 g of C3' were charged in the autoclave to react them under autogenous pressure at a reaction temperature of 85 °C for a reaction time of 1.5 hours (including temperature raising time). The reaction result is shown in Table 1.

Comparative Example 1

Nitrogen heated to 110°C was flowed once at a rate of 1500 ml/minute through the molded body containing the extraction solvent obtained in the same manner as in extraction removal of template of Example 1 to dry the molded body.

The time required for drying was 8 hours. Thereafter, the molded body was taken out from the column, then 4.0 g of the molded body was collected in a glass flask and silylated under heating at 110°C by using 2.7 g of hexamethylsilazane and 40.0 g of toluene. After the liquid was separated by decantation, the molded body was dried at 120°C for 2 hours under vacuum of 10 mmHg to obtain a molded catalyst.

The obtained molded catalyst was evaluated with a batch reaction apparatus in the same manner as in Example 1. The reaction result was shown in Table 1.

Table 1

|  | Example 1 | Comparative Example 1 |
|---|---|---|
| Treatment from extraction to silylation<br>Required time for treatment | Substitution with toluene<br>1 hour and 40 minutes | Drying with hot nitrogen<br>8 hours |
| Reaction result<br>CHPO conversion (%)<br>PO/C3' selectivity (%) *1 | <br>93.2<br>99.1 | <br>92.9<br>98.7 |

*1: PO/C3' selectivity = (produced PO mol/reacted $C_3$' mol) x 100

Industrial Applicability

As described above, according to the present invention, there are provided an efficient process for producing a titanium-containing silicon oxide catalyst which can be used for reaction obtaining, for example, an oxirane compound from a hydroperoxide and an olefin type compound and which can exhibit high activity, and the catalyst obtained by the process.

**Claims**

**1.** A process for producing a titanium-containing silicon oxide catalyst satisfying all of the following conditions (1) to (3);

(1) an average pore diameter is 10Å or more,
(2) 90% or more of the total pore volume has a pore diameter of 5 to 200Å, and
(3) a specific pore volume is 0.2 cm$^3$/g or more,

which comprises the following first to fourth steps:

first step: a step of obtaining a solid containing a catalyst component and a template by mixing and stirring a silica source, a titanium source and a quaternary ammonium ion as a template in a liquid state;
second step: a step of removing the template from the solid obtained in the first step by solvent extraction;
third step: a step of substituting the solvent used for the extraction which was contained in the solid after the removal of the template, with a solvent which is substantially inert to a silylating agent to be used in the following fourth step; and
fourth step; a step of obtaining a silylated catalyst by subjecting the solid obtained in the third step to silylation.

**2.** The process according to claim 1, wherein the solvent for substitution used in the third step is the same as the solvent for silylation used in the fourth step.

**3.** The process according to claim 1 or 2, wherein the template used in the first step is a quaternary ammonium ion represented by the following general formula (I) is used as a template and then the template is removed

$$[NR^1R^2R^3R^4]^+ \qquad\qquad (I),$$

wherein, $R^1$ represents a linear or branched hydrocarbon group having 2 to 36 carbon atoms, and $R^2$ to $R^4$ represent an alkyl group having 1 to 6 carbon atoms.

4. The process according to any one of claims 1 to 3, wherein the process further comprises a step of molding the solid containing the catalyst component.

5. The process according to any one of claims 1 to 4, wherein the solvent for extraction is an alcohol.

6. The process according to claim 5, wherein the alcohol is methanol.

7. The process according to any one of claims 1 to 6, wherein the solvent for substitution is a hydrocarbon.

8. The process according to claim 7, wherein the hydrocarbon is toluene.

9. A titanium-containing silicon oxide catalyst obtained by the process according to any one of claims 1 to 8.

10. A process for producing an oxirane compound, which comprises reacting an olefin type compound with a hydroperoxide in the presence of the catalyst of claim 9.

## INTERNATIONAL SEARCH REPORT

| | International application No. |
|---|---|
| | PCT/JP03/16073 |

**A. CLASSIFICATION OF SUBJECT MATTER**
Int.Cl$^7$ B01J29/89, 35/10, C07D301/19, 303/04

According to International Patent Classification (IPC) or to both national classification and IPC

**B. FIELDS SEARCHED**

Minimum documentation searched (classification system followed by classification symbols)
Int.Cl$^7$ B01J21/00-38/74,, C07D301/19, 303/04

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 2002-239381 A (Sumitomo Chemical Co., Ltd.),<br>27 August, 2002 (27.08.02),<br>Claim 1; Par. No. [0001]<br>& EP 1371417 A1<br>Claim 1; Par. No. [0001]<br>& WO 02/066157 A1 | 9,10<br>1-8 |

☐ Further documents are listed in the continuation of Box C.    ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>10 March, 2004 (10.03.04) | Date of mailing of the international search report<br>23 March, 2004 (23.03.04) |
|---|---|
| Name and mailing address of the ISA/<br>    Japanese Patent Office | Authorized officer |
| Facsimile No. | Telephone No. |

Form PCT/ISA/210 (second sheet) (July 1998)